# EUROPEAN PATENT SPECIFICATION

(11) **EP 3 687 610 B1**
(45) Date of publication and mention of the grant of the patent: **17.01.2024**
(21) Application number: 18778890.6
(22) Date of filing: 25.09.2018
(51) Int. Cl.: A61M 16/04, A63B 71/08, A61C 5/90

(54) **A TOOTH PROTECTOR**
ZAHNSCHUTZ
PROTECTEUR DE DENT

(30) Priority: 25.09.2017 DK PA201770723
(43) Date of publication of application: 05.08.2020
(73) Proprietor: Imed APS, 8300 Odder (DK)
(72) Inventor: BRUHN, Ulrik, Kjelstrøm, 8300 Odder (DK)
(74) Representative: Inspicos P/S
(86) International application number: PCT/EP2018/075961
(87) International publication number: WO 2019/057989

(56) References cited:
- EP-A2- 1 398 061
- WO-A1-2010/038171
- US-A- 5 163 840
- US-A1- 2013 146 066

## Description

### FIELD OF THE INVENTION

The present invention relates to a tooth protector e.g. for use during intubation.

### BACKGROUND

EP1398061A1 discloses an oral appliance suitable for use as mouth guard. The mouth guard comprises a U-shaped base and defining a channel for a row of teeth. Teeth engaging elements formed by EVA encloses and encapsulates the base. This enables it to be shaped to conform to the arch and teeth of a user. The appliance further comprises a notch in an upper surface. The notch permits inward and outward adjustment of the U-shaped member.

During intubation of a patient, special care of the patient's teeth must be taken to avoid pressure on the teeth and gums of the patient. The intubation apparatus may rest against the teeth and possibly cause dental damage. Therefore, there is a need for a tooth protector to avoid such injuries. The tooth protector to be used must be firmly fastened to the teeth to avoid detachment and injuries.

### SUMMARY

It is an object of embodiments of the present disclosure to provide a tooth protector which protects either upper or lower set of teeth of a wearer and which fits snugly to the teeth, which is simple to manufacture, and/or which is safe and easy to sterilize.

In a first aspect, a tooth protector is provided according to claim 1.

When used herein, the term "at least one of x and y" should be understood as x or y or both x and y. I.e. the at least one protrusion could be formed in the facial side or in the lingual side or in both the facial side and the lingual side.

Due to the elastically deformable protrusion, the tooth protector can be maintained in position by elastic deformation of the protrusion against a tooth. This is a very simple structure which facilitates easy and cheap manufacturing and sterilization, and which can provide a good comfort for the user.

The tooth protector forms a cover for either upper or lower dentition of a wearer and may serve to protect the teeth from dental injuries which may occur during e.g. intubation of the wearer. The tooth protector may cover molars, premolars, canines, and incisors of the wearer.

The facial side is a side of the tooth protector facing cheeks and lips of the wearer when mounted onto the teeth. The lingual side is a side of the tooth protector facing the central part of an oral cavity, i.e., when the tooth protector is mounted on the upper teeth, the lingual side faces a hard plate of the oral cavity, while when the tooth protector is mounted on the lower teeth, the lingual side faces a tongue of the wearer. The bottom side of the tooth protector faces the lower set of teeth when the tooth protector is mounted on the upper set of teeth, or it faces the upper set of teeth when the tooth protector is mounted on the lower set of teeth.

The facial and lingual sides are connected via the bottom side so that all three sides together provide a full coverage for the set of teeth. Particularly, the facial, lingual, and bottom sides form a U-shape when seen in a cross section.

The tooth protector further comprises at least one protrusion, which may be formed as an integral part of the facial and/or lingual side, or which may be attached to the facial and/or lingual side, and which protrudes above the surface of one of these sides. The at least one protrusion further extends towards the facial side in case it forms part of the lingual side, and vice versa. Namely, the at least one protrusion protrudes towards the teeth and is in an abutting contact with the teeth. I.e. the facial side and the lingual side both have an inner surface pointing towards the teeth, and the protrusion extends upwardly from one or both of these inner surfaces.

The protrusion being integral part of the facial and/or lingual side means that the protrusion and the side is made in one piece facilitating manufacturing and the ability to handle and sterilise the tooth protector.

The protrusion is elastically deformable to facilitate holding of the tooth protector in a fitted position on the set of teeth by deformation of the protrusion upon contact with the at least one tooth. The elasticity of the protrusion may be achieved by choosing a flexible material to form the protrusion, or by designing the protrusion to have a shape which can provide elastic deformation. Such a shape may include the shape of a protruding cone or dome, and particularly, the elasticity can be provided by reducing the wall thickness of the material to allow easier deformation and/or by use of a material having elastic properties. The protrusion may particularly be configured for at least 50 percent deformation such that the projection away from that one of the lingual or facial sides to which it is attached becomes less than half the length by the elastic deformation, e.g. less than 25 percent of the length of the non-deformed protrusion.

The elastic nature of the protrusion on one of the facial and lingual sides of the tooth protector makes the tooth protector fit snugly to the teeth. While the protrusion on one of the facial and lingual sides abuts the tooth on one side, it may also improve contact between the surface of the other side of the tooth and the inner surface of the other one of the facial and lingual sides of the tooth protector. The protrusion may therefore replace or supplement use of an adhesive substance applied into the tooth protector, as suggested in some of the prior art, and in some embodiments, adhesive may be avoided. Further, a rather simple tooth protector is provided having no additional elements which would serve for fixation of the tooth protector. In this way, there is no restriction on the mouth opening. The wearer may also completely close the mouth if needed.

The facial, lingual and bottom sides may form a substantially U-shaped body. The U-shaped body may be configured to cover a complete set of teeth in the upper or lower part of the mouth, or it may form a section to cover a section of a set of teeth, e.g. to cover one, two, three or four teeth.

The U-shaped body may form a trough in the sense that the facial and lingual sides form the side walls of the trough, and the bottom side forms the bottom of the trough and covers a top surface of the teeth. The U-shaped body is adapted to fit to the shape of the set of teeth. It may be made in different sizes to fit any wearer. It may also be custom made.

The tooth protector may comprise at least two protrusions formed in at least one of the facial side and the lingual side and extending towards the other one of the facial and lingual sides. The at least two protrusions provide a larger force which keeps the tooth protector in a fixed position, compared to the force provided by only one protrusion. The at least two protrusions may be positioned at different places of the U-shaped body.

In one embodiment, at least one protrusion may be formed in one end of the U-shaped body, and at least one protrusion may be formed in another end of the U-shaped body. The protrusions may both be formed in the facial side or they may both be provided in the lingual side. If the protrusions are formed in the facial side, they will create a force directed towards the central part of the oral cavity, keeping the tooth protector in the fixed place. Particularly, the protrusions may be symmetric, meaning that one protrusion constitutes a reflection of the other in a plane of symmetry extending between the two free ends of the U-shaped body.

In another embodiment, at least one protrusion may be formed in one end of the U-shaped body in the facial side, and at least one protrusion may be formed in another end of the U-shaped body in the lingual side. Having protrusions in the opposite sides of the tooth protector provides an increased force which keeps the tooth protector in the fixed place.

In yet another embodiment, at least two protrusions may be formed opposite to each other in one end of the U-shaped body, and at least one protrusion may be formed in the facial side and at least one protrusion may be formed in the lingual side. Therefore, a set of protrusions may provide contact to both sides of at least part of one tooth. Another end of the U-shaped body may be provided with another set of the protrusions, as well as only one protrusion.

The tooth protector further comprises at least one opening through the U-shaped body. The opening is formed at the protrusion. In case of more than one protrusion, each protrusion may have a corresponding opening. The opening may be formed in the bottom side and/or in that one of the facial or lingual side where the protrusion is. The opening is in the shape of a slit, i.e. a narrow oblong opening. Herein, a slit is defined as an opening extending further in one direction than in another direction, preferably having a length being at least 5-10 times the width. The slit may e.g. be formed only in the bottom side of the tooth protector. In another embodiment, the opening may extend from the bottom side and along one or both of the facial and lingual sides, and up to a location at or near the protrusion. The opening may particularly provide the effect that the elastic deformation of the protrusion increases.

The tooth protector may be made of a resilient material, such as thermoplastic rubber such as an elastomer, vulcanized rubber, thermoplastic, copolymer or the like. The resilient material enables elastic deformation of the protrusions, as well as the flexibility of the entire tooth protector. Namely, one size tooth protector may fit a number of wearers having jaws of different sizes.

The protrusion may be in an abutting contact with at least a portion of one tooth. This portion may be a central portion of the tooth, such that only one protrusion abuts the tooth. In another case, a number of protrusions may abut one tooth. Both configurations may provide sufficient force to keep the tooth protector in the fixed position.

The protrusions may have different shapes. In one embodiment, at least one protrusion may be dome-shaped. Dome-shaped protrusion will elastically deform in a direction of the side from which it is formed, will further flatten out, increasing the contact surface with the teeth, ensuring the tooth protector fixation.

At least one protrusion may be in the shape of a pin which may form a 90 degree angle with the one of the facial or lingual side surfaces, i.e. extending perpendicularly upwards from the surface. Herein, that means that a centre axis of the protrusion can be defined, and that centre axis is perpendicular to a surface tangent, i.e. forms a normal to the surface. When the tooth protector is applied, the pin will bend towards the bottom side fixing the tooth protector to the teeth. The pin may have different lengths, but may not be longer than a height of the tooth protector. The pin may be positioned at a top portion of the facial and/or lingual side, further away from the bottom side. The pin may be rectangular, trapezoidal, or the like. Particularly, the pin may have a symmetric shape when seen in a cross section transverse to the centre axis of the pin. By symmetric shape is herein meant that at least one line can be defined in the cross section, thereby forming two identical shapes mirrored in that one line.

A pin forming a flat upper surface may have a larger area of contact with a tooth, and therefore such a flat surface may increase the friction and thus the ability of the pin shaped protrusion to hold the position of the tooth protector. Regardless of the shape of the protrusion, each protrusion may have either its own corresponding opening or a number of protrusions may have one common opening.

Generally, the protrusions may be made such that the size of the protrusion corresponds to the average size of a tooth placed at the corresponding position of the protrusion. In that way, one protrusion may be in contact with the inner or outer surface of that tooth.

The tooth protector may be made in one piece. Namely, all three sides, facial, lingual and bottom sides, as well as one or more protrusions may be made of the same material and fabricated in one step. This increases robustness of the tooth protector.

In one embodiment, the one piece tooth protector may be made by 2-k injection of two different polymer materials. A first polymer material may form the facial, lingual, and bottom sides, while a second polymer material may form one or more protrusions. The first polymer may have larger rigidity than the second. The first polymer material may be a more rigid polymer such as the shaft of a tooth brush, e.g. made of PP, or PE. Namely, the sides of the tooth protector may not be flexible as they only serve as a protection. Another polymer forming the protrusions may be of a resilient polymer to create a flexible contact which can be deformed against a tooth and to hold the tooth protector by elastic deformation. As mentioned above, polymer materials may be selected from a group of elastomers, e.g. like the bendable part of a tooth brush, EPDM etc.

The facial side of the tooth protector may comprise a groove positioned in the central portion of the facial side. Namely, the facial side may have a smaller height at its central portion. The groove enables an easy attachment/detachment of the tooth protector to/from the mouth. Furthermore, it leaves space for the labial frenulum of the wearer.

The deformation of the protrusions may provide a sufficient force keeping the tooth protector in a fixed position. This is important as it prevents an accidental detachment of the tooth protector from the fixed position.

In an aspect not according to the invention, a method is described by which a tooth protector according to the first aspect is attached to the teeth of a person by elastic deformation of the at least one protrusion against a tooth.

In a second aspect, a set of different tooth protectors according to the first aspect and having different sizes, shapes, or different layout of protrusions and/or openings is provided.

The set may enable the user to compare and find one tooth protector which provides the best fit on the wearer.

### BRIEF DESCRIPTION OF THE DRAWINGS

The invention will now be described in further details with reference to the accompanying drawings in which:
Fig. 1 illustrates a perspective view of a tooth protector according to one embodiment of the invention,
Fig. 2a illustrates a perspective view of a tooth protector according to another embodiment of the invention,
Fig. 2b illustrates an enlarged view of an upper surface of protrusions in the form of projecting pins,
Figs. 3a and 3b illustrate perspective views of embodiments, and
Fig. 4 illustrates a cross section, a slot opening increasing the ability of the protrusion to deflect.

### DETAILED DESCRIPTION OF THE DRAWINGS

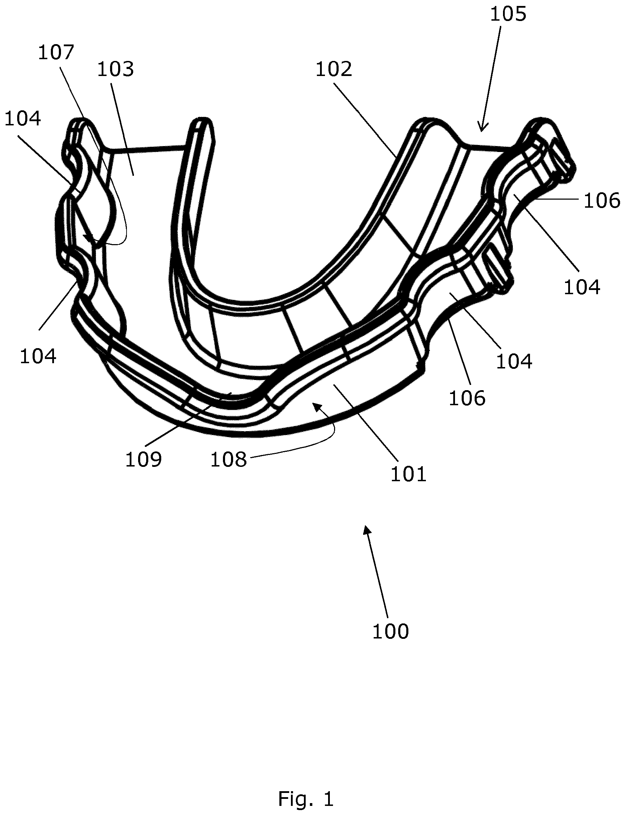

Fig. 1a is a perspective view of a tooth protector 100 according to one embodiment of the invention. The tooth protector 100 has a facial side 101, a lingual side 102, and a bottom side 103. The facial side 101 faces cheeks and lips of the wearer when mounted onto the teeth. The lingual side 102 faces the central part of an oral cavity, i.e., when the tooth protector 100 is mounted on the upper teeth, the lingual side 102 faces a hard plate of the oral cavity, while when the tooth protector 100 is mounted on the lower teeth, the lingual side 102 faces a tongue of the wearer. The bottom side 103 of the tooth protector 100 faces the lower set of teeth when the tooth protector 100 is mounted on the upper set of teeth, or it faces the upper set of teeth when the tooth protector 100 is mounted on the lower set of teeth.

The facial side 101 and the lingual side 102 are connected via the bottom side 103 so that all three sides together are therefore configured to provide a full coverage for the set of teeth, either lower or upper.

The tooth protector 100 shown in Fig. 1 further comprises four protrusions 104. In the embodiment illustrated in Fig. 1, the protrusions are formed by the shape of the entire wall of the facial side. It could likewise be protrusions formed by the shape of the entire wall of the lingual side 102. By this design, attaching the protrusions as separate components is unnecessary, and manufacturing and handling becomes easier. In the specific embodiment, the protrusions, while projecting inwardly from the inner surface 107, i.e. the surface pointing towards the teeth, also define an indentation in the outer surface 108, i.e. the surface pointing away from the teeth. The protrusions form an integral part of the facial side 101 and protrude beyond the normal line surface of the facial side 101. The four protrusions 104 extend towards the lingual side 102. Each of the four protrusions 104 protrudes towards at least one tooth and will be in an abutting contact with the at least one tooth.

The protrusions 104 are elastically deformable to facilitate holding of the tooth protector 100 in a fitted position on the set of teeth by deformation of the protrusions 104 upon contact with the at least one tooth. The elasticity of the protrusions 104 shown in Fig. 1 is achieved by designing the shape of protrusions 104 to provide elastic deformation, but it may as well be achieved by choosing a flexible material to form the protrusions 104.

Based on the elasticity of the protrusions 104, the tooth protector 100 will fit snugly to the teeth as it abuts the tooth on the facial side 101, and at the same time increases the force on the contact surface between the lingual side of the tooth and the lingual side 102 of the tooth protector 100, regardless of movements of a jaw. Having such means for stabilization of the tooth protector 100, a rather simple tooth protector 100 is provided having no additional elements which would serve for fixation of the tooth protector 100.

The facial side 101, lingual side 102, and bottom side 103 form a substantially U-shaped body. The U-shaped body forms a trough 105 having the facial 101 and lingual 102 sides as side walls which embrace a crown of teeth, while the bottom side 103 of the U-shaped body covers a top surface of the teeth. Two protrusions 104 on the opposite ends of the U-shaped body will abut two premolars, while the other two may abut two molars. Their size may correspond to sizes of molars and premolars.

The tooth protector 100 further comprises slits 106 at each of the four protrusions (only two are shown). The slits are formed in the bottom side 103. By having the slits 106, the elastic deformation of the protrusions 104 is further enabled. Fig. 1b illustrates the slits.

The tooth protector 100 shown in Fig. 1a and 1b is made in one piece. Namely, all three sides, facial, lingual and bottom sides, 101, 102, and 103, as well as four protrusions 104 are made of the same material and fabricated in one step.

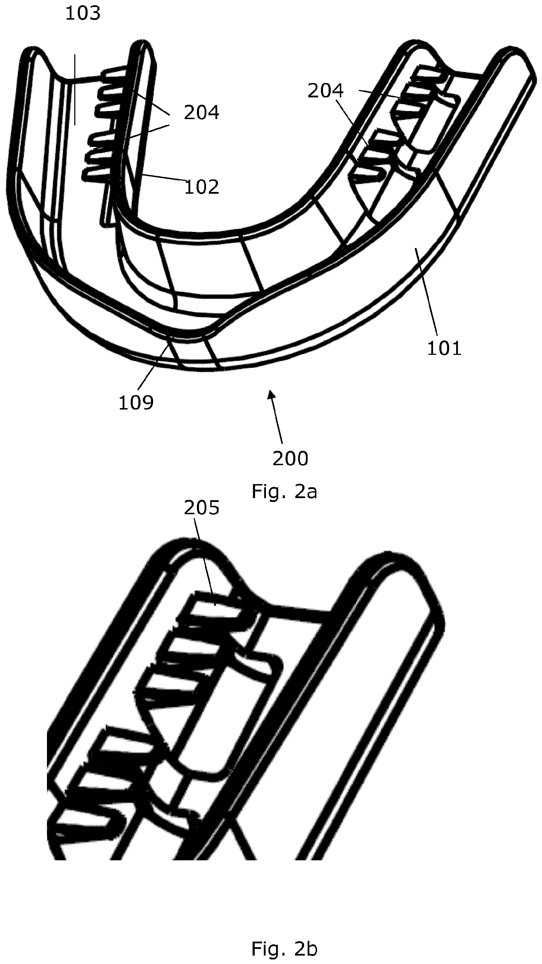

Fig. 2a is a perspective view of a tooth protector 200 according to another embodiment of the invention. As in the previous embodiment shown in Fig. 1a and 1b, the tooth protector 200 has a facial side 101, a lingual side 102, and a bottom side 103.

The tooth protector 200 shown in Fig. 2a further comprises four groups of protrusions, each group comprising 3 protrusions 204 having a shape of a pin, which form an integral part of the lingual side 102, or which are separate parts being attached to the lingual side. The pins protrude beyond the normal line surface of the lingual side 102. The twelve protrusions 204 extend towards the facial side 101. Each group of three protrusions 204 protrudes towards at least one tooth, and all the three protrusions 204 of one group will be in an abutting contact with the at least one tooth. The pins 204 form an angle in the range of 90 degree with the lingual side 102. When the tooth protector 200 is applied, the pins 204 will bend towards the bottom side 103, fixing the tooth protector 200 to the teeth. The pins 204 may have different lengths, but may not be longer than a height of the tooth protector 200. The pins 204 are positioned at a top portion of the lingual side 102, further away from the bottom side 103. The pins have flat upper surfaces to increase the friction against a tooth. The flat upper surface 205 is illustrated in the enlarged view in Fig. 2b.

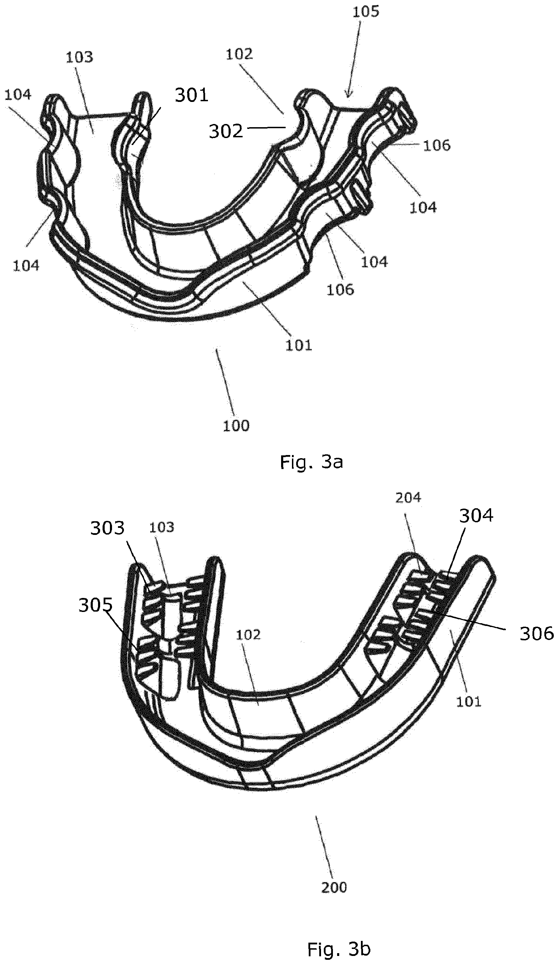

Fig. 3a illustrates a perspective view of a tooth protector 100 similar to the one illustrated in Fig. 1, but having protrusions 301, 302 in the lingual side 102. These protrusions are illustrated in addition to the four protrusions 104 in the facial side 101, however, they could also replace the four protrusions in the facial side. The illustrated two protrusions 301, 302 are one example of a configuration of the lingual side. Other numbers, e.g. 3, 4, 5, 6 protrusions could be provided in the lingual side, with or without further protrusions in the facial side.

Fig. 3b is a perspective view of a tooth protector 200 corresponding to the tooth protector illustrated in Figs. 2a and 2b.

The tooth protector illustrated in Fig. 3b further comprises four additional groups 303, 304, 305, 306 of pin shaped protrusions which is attached to the facial side 101. The pins protrude beyond the normal line surface of the facial side 101. The twelve protrusions extend towards the lingual side 102. The flat upper surfaces of the additional pin shaped protrusions facilitate increased friction resistance against teeth. In general, one or more groups of a varying number of pins could be located on the facial and/or the lingual side.

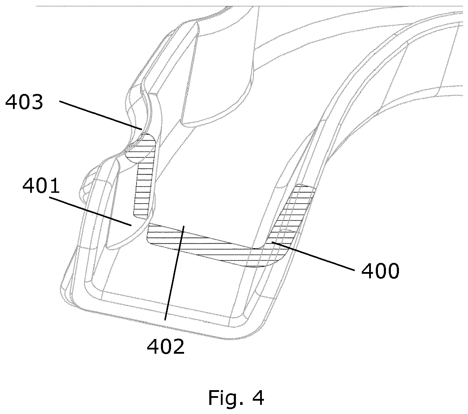

Fig. 4 illustrates a cross section 400 of the embodiment illustrated in Fig. 1. In this view, it is clearly seen that an opening 401 is formed in the area where the bottom side 402 joins the protrusion 403. The slit forming the opening increases the ability of the body to deform elastically by decoupling the protrusion from the bottom portion.

## Claims

1. A tooth protector (100) forming a facial side (101), a lingual side (102), and a bottom side (103), the facial, lingual and bottom sides configured to provide coverage for a set of teeth, wherein the tooth protector further comprises at least one protrusion (104, 204, 205, 403) formed in at least one of the facial side and the lingual side and extending towards the other one of the facial and lingual sides, the protrusion being elastically deformable to facilitate holding of the tooth protector in a fitted position on the set of teeth by deformation of the protrusion upon contact with a tooth, the tooth protector further comprises at least one opening (106, 401) through the U-shaped body, the opening increasing the ability of the body to deform elastically, **characterised in that** the at least one protrusion (104, 204, 205, 403) projects inwardly from an inner surface (107) of the facial side or lingual side and forms an indentation in an opposite outer surface (108) of the facial or lingual side and the opening forms the shape of a slit (106) extending between the bottom side (103, 402) and one of the at least one protrusions. (104, 403).

2. A tooth protector according to any of the preceding claims, comprising at least two protrusions formed in at least one of the facial side and the lingual side and extending towards the other one of the facial and lingual sides.

3. A tooth protector according to any of the preceding claims, wherein the facial, lingual, and bottom sides form a substantially U-shaped body, the U-shaped body configured to cover a complete set of teeth in the upper or lower part of the mouth, and wherein at least one protrusion is formed in one end of the U-shaped body, and at least one protrusion is formed in another end of the U-shaped body, both protrusions being formed in the same one of the facial side and the lingual side.

4. A tooth protector according to any of the preceding claims, wherein the at least one protrusion is dome-shaped.

5. A tooth protector according to any of the claims 1-3, wherein the at least one protrusion is in the shape of a pin, the pin defining a centre axis extending as a normal to the surface of the facial or lingual side.

6. A tooth protector according to any of the preceding claims, wherein the at least one protrusion is positioned in an area of a premolar or molar tooth when the tooth protector is attached to a set of teeth.

7. A tooth protector according to any of the preceding claims, wherein the facial side comprises a groove (109) positioned in the central portion of the facial side.

8. A tooth protector according to any of the preceding claims, wherein the facial side (101), the lingual side (102), the bottom side, and the at least one protrusion is made in one piece.

9. A set comprising a plurality of tooth protectors according to any of claims 1-8, each tooth protector differing from other tooth protectors at least with respect to one of:
• the size or shape of the facial side, the lingual side, or the bottom side,
• the size or shape of protrusions,
• the size or shape of openings,
• the layout of the protrusions, or
• the layout of the openings.

## Patentansprüche

1. Zahnschutz (100), der eine Gesichtsseite (101), eine Zahninnenseite (102) und eine Unterseite (103) bildet, wobei die Gesichtsseite, die Zahninnenseite und die Unterseite dazu konfiguriert sind, eine Abdeckung für eine Zahnreihe bereitzustellen, wobei der Zahnschutz ferner mindestens einen Vorsprung (104, 204, 205, 403) umfasst, der in mindestens einer der Gesichtsseite und der Zahninnenseite ausgebildet ist und sich in Richtung der anderen der Gesichtsseite und der Zahninnenseite erstreckt, wobei der Vorsprung elastisch verformbar ist, um das Halten des Zahnschutzes in einer angepassten Position auf der Zahnreihe durch Verformung des Vorsprungs bei Kontakt mit einem Zahn zu erleichtern, wobei der Zahnschutz ferner mindestens eine Öffnung (106, 401) durch den U-förmigen Körper umfasst, wobei die Öffnung die Eigenschaft des Körpers, sich elastisch zu verformen, verstärkt, **dadurch gekennzeichnet, dass** der mindestens eine Vorsprung (104, 204, 205, 403) von einer Innenfläche (107) der Gesichtsseite oder Zahninnenseite nach innen hervorsteht und eine Vertiefung in einer gegenüberliegenden Außenfläche (108) der Gesichtsseite oder Zahninnenseite bildet und die Öffnung die Form eines Schlitzes (106) bildet, der sich zwischen der Unterseite (103, 402) und einem der mindestens einen Vorsprünge (104, 403) erstreckt.

2. Zahnschutz nach einem der vorhergehenden Ansprüche, umfassend mindestens zwei Vorsprünge, die in mindestens einer der Gesichtsseite und der Zahninnenseite ausgebildet sind und sich in Richtung der anderen der Gesichtsseite und der Zahninnenseite erstrecken.

3. Zahnschutz nach einem der vorhergehenden Ansprüche, wobei die Gesichtsseite, die Zahninnenseite und die Unterseite einen im Wesentlichen U-förmigen Körper bilden, wobei der U-förmige Körper dazu konfiguriert ist, eine komplette Zahnreihe im oberen oder unteren Teil des Mundes abzudecken, und wobei mindestens ein Vorsprung in einem Ende des U-förmigen Körpers ausgebildet ist und mindestens ein Vorsprung in einem anderen Ende des U-förmigen Körpers ausgebildet ist, wobei beide Vorsprünge in der gleichen von der Gesichtsseite und der Zahninnenseite ausgebildet sind.

4. Zahnschutz nach einem der vorhergehenden Ansprüche, wobei der mindestens eine Vorsprung kuppelförmig ist.

5. Zahnschutz nach einem der Ansprüche 1-3, wobei der mindestens eine Vorsprung die Form eines Stifts aufweist, wobei der Stift eine Zentralachse definiert, die sich als Normale zur Oberfläche der Gesichtsseite oder Zahninnenseite erstreckt.

6. Zahnschutz nach einem der vorhergehenden Ansprüche, wobei der mindestens eine Vorsprung in einem Bereich eines Prämolaren oder Molaren positioniert ist, wenn der Zahnschutz an einer Zahnreihe angebracht ist.

7. Zahnschutz nach einem der vorhergehenden Ansprüche, wobei die Gesichtsseite eine Rille (109) umfasst, die im zentralen Abschnitt der Gesichtsseite positioniert ist.

8. Zahnschutz nach einem der vorhergehenden Ansprüche, wobei die Gesichtsseite (101), die Zahninnenseite (102), die Unterseite und der mindestens eine Vorsprung in einem Stück hergestellt wird.

9. Satz, umfassend eine Vielzahl von Zahnschützern nach einem der Ansprüche 1-8, wobei jeder Zahnschutz sich von anderen Zahnschützern zumindest in Bezug auf eines von Folgendem unterscheidet:
• die Größe oder Form der Gesichtsseite, der Zahninnenseite oder der Unterseite,
• die Größe oder Form der Vorsprünge,
• die Größe oder Form der Öffnungen,
• die Auslegung der Vorsprünge oder
• die Auslegung der Öffnungen.

## Revendications

1. Protège-dent (100) formant un côté facial (101), un côté lingual (102) et un côté inférieur (103), les côtés facial, lingual et inférieur étant configurés pour fournir une couverture à une denture, dans lequel le protecteur dentaire comprend en outre au moins une protubérance (104, 204, 205, 403) formée dans au moins un parmi le côté facial et le côté lingual et s'étendant vers l'autre des côtés facial et lingual, la protubérance étant élastiquement déformable pour faciliter la tenue du protège-dent dans une position ajustée sur la denture par déformation de la protubérance lors du contact avec une dent, le protège-dent comprend en outre au moins une ouverture (106, 401) à travers le corps en forme de U, l'ouverture augmentant la capacité du corps à se déformer élastiquement, **caractérisé en ce que** au moins une protubérance (104, 204, 205, 403) dépasse (107) du côté facial ou côté lingual et forme une indentation dans une surface externe opposée (108) du côté facial ou lingual et l'ouverture forme la forme d'une fente (106) s'étendant entre le côté inférieur (103, 402) et une de la ou des protubérances (104, 103) .

2. Protège-dent selon une quelconque des revendications précédentes, comprenant au moins deux protubérances formées dans au moins un du côté facial et du côté lingual et s'étendant vers l'autre des côtés facial et lingual.

3. Protège-dent selon une quelconque des revendications précédentes, dans lequel les côtés facial, lingual et inférieur forment un corps sensiblement en forme de U, le corps en forme de U étant configuré pour recouvrir une denture complète dans la partie supérieure ou inférieure de la bouche et dans lequel au moins une protubérance est formée dans une extrémité du corps en forme de U, et au moins une protubérance est formée dans une autre extrémité du corps en forme de U, les deux protubérances étant formées dans la même du côté facial et du côté lingual.

4. Protège-dent selon une quelconque des revendications précédentes, dans lequel la au moins une protubérance est en forme de dôme.

5. Protège-dent selon une quelconque des revendications 1 à 3, dans lequel la au moins une protubérance a la forme d'une broche, la broche définissant un axe central s'étendant normalement à la surface du côté facial ou lingual.

6. Protège-dent selon une quelconque des revendications précédentes, dans lequel la au moins une protubérance est positionnée dans une zone d'une prémolaire ou d'une molaire lorsque le protecteur de dent est fixé à une denture.

7. Protège-dent selon une quelconque des revendications précédentes, dans lequel le côté facial comprend une rainure (109) positionnée dans la partie centrale du côté facial.

8. Protège-dent selon une quelconque des revendications précédentes, dans lequel le côté facial (101), le côté lingual (102), le côté inférieur et la au moins une protubérance sont réalisés en un seul tenant.

9. Ensemble comprenant une pluralité de protège-dents selon une quelconque des revendications 1 à 8, chaque protège-dent différant des autres protecteurs de dents au moins par rapport à un des éléments suivants :
• la taille ou la forme du côté facial, du côté lingual, ou du côté inférieur,
• la taille ou la forme des protubérances,
• la taille ou la forme des ouvertures,
• la disposition des protubérances ou
• la disposition des ouvertures.
